**Europäisches Patentamt**

(19) **European Patent Office**

**Office européen des brevets**

(11) Publication number : **0 307 400 B1**

(12) **EUROPEAN PATENT SPECIFICATION**

(45) Date of publication of patent specification :
08.04.92 Bulletin 92/15

(51) Int. Cl.⁵ : **A61K 7/32, A61K 31/235**

(21) Application number : **87902669.8**

(22) Date of filing : **12.05.87**

(86) International application number :
**PCT/GB87/00323**

(87) International publication number :
**WO 87/06827 19.11.87 Gazette 87/25**

(54) INHIBITORS OF ESTERASE-PRODUCING MICRO-ORGANISMS, FOR USE PRIMARILY IN DEODORANT COMPOSITIONS.

The file contains technical information submitted after the application was filed and not included in this specification

(30) Priority : **13.05.86 GB 8611650**

(43) Date of publication of application :
**22.03.89 Bulletin 89/12**

(45) Publication of the grant of the patent :
**08.04.92 Bulletin 92/15**

(84) Designated Contracting States :
**AT BE CH DE FR GB IT LI LU NL SE**

(56) References cited :
**EP-A- 0 039 857**

(56) References cited :
**EP-A- 0 054 174**
**BE-A- 458 964**
**DE-A- 2 617 817**
**FR-A- 2 374 039**
**FR-A- 2 374 292**
**FR-A- 2 390 160**
**US-A- 3 808 319**

(73) Proprietor : **ROBERTET S.A.**
**37, avenue Sidi-Brahim**
**F-06130 Grasse (FR)**

(72) Inventor : **BETTS, John, Adrian**
**Valhalla New Road**
**Haslemere Surrey GU27 3RW (GB)**

(74) Representative : **Dixon, Donald Cossar et al**
**Gee & Co. Chancery House Chancery Lane**
**London WC2A 1QU (GB)**

EP 0 307 400 B1

## Description

This invention relates to inhibitors of esterase-producing micro-organisms for use primarily in deodorant compositions.

The human skin as a large natural population of micro-organisms. These organisms are nourished by various skin secreted substances, skin cell debris, breakdown products of the skin and the organisms themselves. The skin secretions are conveniently divided into two groups, water- and lipid-soluble materials; these are eccrine and apocrine sweat and sebum which will be referred to as 'liquid body-secretions' and will now be described, as will their functions as are generally understood.

Eccrine sweat consists mainly of a watery solution of dissolved salts and is produced by glands distributed over the whole skin surface. In conditions of occlusion, e.g. feet enclosed in socks and shoes, the eccrine sweat accumulates and in these warm, damp conditions, the skin debris, together with nutrients from the sweat, provide a medium for micro-organism growth with the possibility of massive overgrowth of one type. This can result, in the first instance, in odorous metabolic products, and in the second, in clinical infection with maceration of the skin and irritation.

Apocrine sweat is produced by the apocrine glands at specific sites on the body, notably the axillae, the anogenital area and around the nipples. Although present at birth, the apocrine glands are not functional until puberty when they are influenced by circulating androgens. Apocrine secretion differs from eccrine sweat in containing lipids (fatty materials) and proteins. In the warm,damp occlusion met in the axilla, certain skin micro-organism metabolise this secretion, forming free fatty acids and other breakdown products. These. materials are odorous and responsible for 'body odour'.

The sebaceous glands are distributed over the skin surface except the palms and dorsae. They are most numerous on the scalp, forehead, face, back and chest. The secretion, sebum, consists mainly of fatty materials, wax esters, cholesterol and its esters and squalene. Normally, sebum flows freely from the glands, spreading over the skin surface. In acneic and certain other skin conditions, the sebaceous duct through which the sebum is normally secreted becomes hyperkeratinised and the opening of the duct becomes blocked. The gland continues to produce sebum and the blocked duct distends to form a comedone. Also blocked in the duct, (the normally) commensal micro-organisms produce esterases which hydrolyse the sebum lipids, liberating free fatty acids. These fatty acids are irritant and can result in an inflammatory reaction along the wall of the duct. Leucocytes invade the inflamed area and the comedone develops into papule and then a pustule. This is a typical acne 'spot'.

The scalp is well supplied with sebaceous glands, and the scalp, like all skin, undergoes desquamation. Due to the presence of hair, the squames tend to be retained at the scalp surface. Sebum accumulates beneath these squames and in dandruff conditions is hydrolysed by micro-organism produced esterases to form irritant fatty acids. The irritation causes proliferation of the epidermis and increased formation of the stratum corneum which again desquamates unevenly in large clumps - the dandruff scale or flake.

EP-A-0 039 857 discloses a deodorant composition comprising an aromatic ester of an alkyl alcohol having from 1 to 10 carbon atoms. As such the deodorant composition will necessarily have a simply destructive and transient effect.

B-A-458 964 discloses a composition containing benzyl benzoate or benzyl cinnamate and a sulphonated sc. sulphated) alcohol as a foaming agent and intended for cleansing the skin and removing make-up. The foaming agent is known to have an irritant effect if allowed to remain on the skin.

It is an object of the present invention to provide a personal deodorant having a formulation which produces effective action over a substantial period of time, which is safe in application, and which is economical and safe to produce.

To fulfil this object, there is provided according to a principal aspect of the present invention, the use in, or in the manufacture of, a deodorant composition, as an inhibitor of esterase-producing micro-organisms, an aromatic acid ester of a phenol or of an aromatic alcohol, the phenol of aromatic alcohol being sufficiently water-soluble to impart an anti-microbial action and/or to lower the pH of liquid body-secretion to a level which at least inhibits the growth of micro-organisms in the liquid body-secretions. The deodorant composition normally also contains a perfume composition, and a vehicle such as ethanol for the aromatic acid ester and the perfume composition. Where a dermatological action is required the vehicle may contain for example a polyol or dimethyl sulphoxide which may also act as a skin penetrant.

The effect of the active ingredient is produced by the aforementioned microbial enzymes acting to split the constituents of the ester and so hydrolyse the ester back into the aromatic acid and the phenol or aromatic alcohol. On a skin surface, such as in deodorant applications, this action occurs almost immediately but where skin penetration is involved, as in most dermatological applications, the action is progressive.

The above term 'anti-microbial action' means an action which inhibits microbial growth, rather than one which eliminates microbial growth completely as can be achieved by a microbicide. In such skin-surface and skin-penetrating applications, the esterases produced by the micro-organism hydrolyse a portion of the active ingredient and, in so doing, inhibit the action

2

of the esterase and further growth of the micro-organism. After a period of time, the micro-organism may resume its metabolic activity and the above-described process is repeated, and repetition will occur until the active ingredient is used up.

'Phenols' are, generally, aromatic compounds containing one or more hydroxyl groups directly attached to a benzene nucleus, but the phenols of the present invention may be restricted to those in which the other positions available on the benzene nucleus are more or less occupied by hydrogen, hydroxyl, aliphatic, benzenoid or heterocyclic groups. Examples of such phenols useful in the present invention include phenol, cresols, xylenols, thymol, carvacrol, eugenol and isoeugenol.

'Aromatic alcohols' are, generally, aromatic compounds with a hydroxyl group in a side chain attached to a benzene nucleus. Again, for the present invention, it is preferable that the other positions available on the benzene nucleus are more or occupied by hydrogen, hydroxyl, aliphatic, benzenoid or heterocyclic groups. Suitable examples include benzyl alcohol, phenylethyl alcohol, cinnamic alcohol and anisic alcohol.

'Aromatic acids' are, generally, compounds containing one or more carboxylic groups which are directly attached to a benzene nucleus or occur in a side chain. Yet again, it is preferable that the other positions available on the benzene nucleus are occupied by hydrogen hydroxyl, aliphatic, benzenoid or heterocyclic groups. Suitable examples include benzoic acid, salicylic acid, cinnamic acid, phenylacetic and anisic acid.

The inhibitor according to the invention incorporates a microbial-inhibiting agent as opposed to the more usual triclosan bactericide which acts to eliminates rather than control the relevant microflora. Known microbicides and bactericides are usually powerful, and it is believed that the complete elimination of microflora, specifically cutaneous flora, is medically undesirable.

Embodiments of the present invention will now be described, by way of example.

As a deodorant for use in an aerosol spray or mechanical spray container, about 5% to 50% and preferably 20% of active ingredient of the invention is incorporated in a perfume composition, and about 1% of the perfume composition is added to a 96% ethanol excipient. The perfume composition of the invention will frequently be sold to an end-producer who will add the composition to his chosen vehicle.

Anti-inflammatory substances such as hydrocortisone or glycyrrhetic acid and healing agents such as allantoin, may also be incorporated in the end product.

As a powder for the treatment of tinea pedis and foot odour, active ingredient (if liquid), within the above percentages, is adsorbed onto amorphous silica powder or light magnesium carbonate which is then mixed with say 50% talcum, starch or other suitable powder. If the active ingredient is solid, usually crystalline, the crystals are finely ground, for example in a microniser, and then mixed with say 50% talcum, starch or other suitable powder.

Suitable perfume compositions may also be incorporated in the deodorant compositions.

The deodorant composition may be supplied in sprinkler bottles in the form of liquid droplets. Alternatively, the lotion may be applied by means of a pad or compress which is pre-impregnated and supplied in a sealed package; the pad is partially exposed and then applied to skin area, at least once per day.

## Claims

1. The use, in the manufacture of a deodorant composition for inhibiting esterase producing micro-organisms present on human skin, of an aromatic acid ester of a phenol or of an aromatic alcohol, the phenol or aromatic alcohol being sufficiently water-soluble to impart an anti-microbial action and the aromatic acid being sufficiently water-soluble to impart an anti-microbial action and/or to lower the pH of liquid body-secretion to a level which at least inhibits the growth of micro-organisms in the liquid body-secretions.

2. The use, as an inhibitor of esterase producing micro-organisms in a deodorant composition, of an aromatic acid ester of a phenol or of an aromatic alcohol, the phenol or aromatic alcohol being sufficiently water-soluble to impart an anti-microbial action and the aromatic acid being sufficiently water-soluble to impart an anti-microbial action and/or to lower the pH of liquid body-secretion to a level which at least inhibits the growth of micro-organisms in the liquid body-secretions, the deodorant composition additionally comprising a perfume composition and a vehicle for said aromatic acid ester and the perfume composition.

3. A deodorant composition consisting of an inhibitor of esterase producing micro-organisms in which the active ingredient consisting of an aromatic acid ester of a phenol or of an aromatic alcohol, the phenol or aromatic alcohol being sufficiently water-soluble to impart an anti-microbial action and the aromatic acid being sufficiently water-soluble to impart an anti-microbial action and/or to lower the pH of liquid body-secretion to a level which at least inhibits the growth of micro-organisms in the liquid body-secretions, a perfume composition, and a vehicle for the active ingredient and perfume composition.

4. Use as claimed in Claim 1 or 2, or a composition as claimed in Claim 3, wherein one or more of the available positions on the benzene nucleus of said phenol are occupied by hydroxyl, aliphatic, benzenoid or heterocyclic groups, and said phenol is preferably selected from phenol, cresols, xylenols, thymol, carvacrol, eugenol and isoeugenol.

5. Use as claimed in Claim 1, 2, or a composition as claimed in Claim 3, wherein one or more of the available positions on the benzene nucleus of the aromatic alcohol are occupied by hydroxyl, aliphatic, benzenoid or heterocyclic groups, and said aromatic alcohol is preferably selected from benzyl alcohol, phenylethyl alcohol, cinnamic alcohol and anisic alcohol.

6. Use as claimed in Claim 1, 2, 4 or 5 or a composition as claimed in Claim 3, 4 or 5, wherein one or more of the available positions on the benzene nucleus of said aromatic acid are occupied by hydroxyl, aliphatic, benzenoid or heterocyclic group, and said aromatic acid is preferably selected from benzoic acid, salicylic acid, cinnamic acid, phenylacetic acid and anisic acid.

7. Use as claimed in Claim 2, or in claims 4, 5 or 6 as appendant thereto or a composition as claimed in Claim 3, 4, 5 or 6, wherein said vehicle comprises an alcohol.

8. Use or a composition as claimed in Claim 7, wherein said aromatic acid ester is present in an amount of 5%-50% in said perfume composition, and said aromatic acid ester and said perfume composition constitute approximately 1% of said composition, the vehicle being approximately 96% ethanol excipient.

9. Use as claimed in any one of Claims 1, or in claims 4, 5 or 6 as appendant thereto or a composition as claimed in any one of claims 3 to 6, wherein said aromatic acid ester is incorporated in a vehicle which comprises aqueous alcohol and/or skin penetrants to produce also a dermatological action.

10. Use or a composition as claimed in Claim 9, and further incorporating anti-inflammatory substances and/or healing agents.

**Patentansprüche**

1. Der Gebrauch, bei der Herstellung eines desodorierenden Präparats für die Hemmung von auf menschlicher Haut vorhandenen Esterase erzeugenden Mikroorganismen, eines aromatischen Säureesters eines Phenols oder eines aromatischen Alkohols, wobei das Phenol oder der aromatische Alkohol genügend wasserlöslich ist, um eine antimikrobielle Wirkung zu vermitteln, und die aromatische Säure genügend wasserlöslich ist, um eine antimikrobielle Wirkung zu vermitteln und/oder den ph-Wert flüssiger Körpersekretion auf einen Grad abzusenken, der mindestens das Wachstum von Mikroorganismen in den flüssigen Körpersekretionen hemmt.

2. Der Gebrauch, als Hemmstoff für Esterase erzeugende Mikroorganismen in einem desodorierenden Präparat, eines aromatischen Säureesters eines Phenols oder eines aromatischen Alkohols, wobei das Phenol oder der aromatische Alkohol genügend wasserlöslich ist, um eine antimikrobielle Wirkung zu vermitteln, und die aromatische Säure genügend wasserlöslich ist, um eine antimikrobielle Wirkung zu vermitteln und/oder den ph-Wert der flüssigen Körpersekretion auf einen Grad abzusenken, der mindestens das Wachstum von Mikroorganismen in den flüssigen Körpersekretionen hemmt, während das desodorierende Präparat zusätzlich ein Duftpräparat und einen Träger für besagten aromatischen Säureester und das Duftpräparat umfaßt.

3. Ein in einem Hemmstoff für Esterase erzeugende Mikroorganismen bestehendes Präparat, bestehend in einem Wirkstoff in der Form eines aromatischen Säureesters eines Phenols oder eines aromatischen Alkohols, wobei das Phenol oder der aromatische Alkohol genügend wasserlöslich ist, um eine antimikrobielle Wirkung zu vermitteln, und die aromatische Säure genügend wasserlöslich ist, um eine antinikrobielle Wirkung zu vermitteln und/oder den ph-Wert der flüssigen Körpersekretion auf einen Grad abzusenken, der mindestens das Wachstw von Mikroorganismen in den flüssigen Körpersekretionen hemmt, sowie in einem Duftpräparat und einem Träger für den Wirkstoff und das Duftpräparat.

4. Gebrauch nach Anspruch 1 oder 2 oder ein Präparat nach Anspruch 3, bei dem eine oder mehrere der verfügbaren Positionen am Benzolkern des besagten Phenols von Hydroxyl-, aliphatischen, benzolartigen oder heterozyklischen Gruppen eingenommen werden und das besagte Phenol vorzugsweise aus der Gruppe Phenol, Kresole, Xylenole, Thynol, Carvacrol, Eugenol und Isoeugenol ausgewählt ist.

5. Gebrauch nach Anspruch 1 oder 2 oder ein präparat nach Anspruch 3, bei dem eine oder mehrere der verfügbaren Positionen am Benzolkern des aromatischen Alkohols von Hydroxyl-, aliphatischen, benzolartigen oder heterozyklischen Gruppen eingenommen werden und der besagte aromatische Alkohol vorzugsweise aus der Gruppe Benzylalkohol, Phenyläthylalkohol, Zimtalkohol und Anisalkohol ausgewählt ist.

6. Gebrauch nach Anspruch 1, 2, 4 oder 5 oder ein Präparat nach Anspruch 3, 4 oder 5, bei dem eine oder mehrere der verfügbaren Positionen am Benzolkern der besagten aromatischen Säure durch Hydroxyl-, aliphatische, benzolartige oder heterozyklische Gruppen eingenommen werden, und die besagte aromatische Säure vorzugsweise aus der Gruppe Benzolsäure, Salicylsäure, Zimtsäure, phenylessigsäure und Anissäure ausgewählt ist.

7. Gebrauch nach Anspruch 2 oder nach Ansprüchen 4, 5 oder 6 wie hier beigefügt oder ein Präparat nach Anspruch 3, 4, 5 oder 6, bei dem der besagte Träger einen Alkohol umfaßt.

8. Gebrauch oder ein präparat nach Anspruch 7, bei dem der besagte aromatische Säureester in einer Konzentration von 5%-50% in dem besagten Duftpräparat vorhanden ist, und der besagte aromatische Säureester und das besagte Duftpräparat etwa 1% des besagten präparats ausmachen, wobei der Träger zu etwa 96% Äthanolträger ist.

9. Gebrauch nach Anspruch 1 oder nach den Ansprüchen 4, 5 oder 6, wie hier beigefügt, oder ein Präparat nach einem der Ansprüche 3 bis 6, bei dem der besagte aromatische Säureester in einen wässerigen Alkohl und/oder zwecks zusätzlicher Bewirkung eines dermatologischen Effekts auch Penetrationsstoffe umfassenden Träger einverleibt ist.

10. Gebrauch eines Präparats nach Anspruch 9 unter zusätzlicher Einverleibung von entzündungshemmenden Stoffen und/oder heilenden Mitteln.

## Revendications

1. Utilisation, dans la fabrication d'une composition déodorante conçue pour inhiber les micro-organismes producteurs d'estérases présents sur la peau humaine, d'un ester acide aromatique d'un phénol ou d'un alcool aromatique, le phénol ou l'alcool aromatique étant suffisamment hydrosoluble pour conférer une action anti-microbienne et l'acide aromatique étant suffisamment hydrosoluble pour conférer une action anti-microbienne et/ou pour abaisser le pH d'une sécrétion corporelle liquide à un niveau qui empêche au moins la croissance des micro-organismes dans les sécrétions corporelles liquides.

2. Utilisation, en tant qu'inhibiteur de micro-organismes producteurs d'estérases dans une composition déodorante, d'un ester acide aromatique ou d'un phénol ou d'un alcool aromatique, le phénol ou l'alcool aromatique étant suffisamment hydrosoluble pour conférer une action anti-microbienne et l'acide aromatique étant suffisamment hydrosoluble pour conférer une action anti-microbienne et/ou pour abaisser le pH de la sécrétion corporelle liquide à un niveau qui empêche au moins la croissance des micro-organismes dans les sécrétions corporelles liquides, la composition déodorante comportant en outre une composition parfumée et un véhicule pour lesdits ester acide aromatique et composition parfumée.

3. Composition déodorante comprenant un inhibiteur de micro-organismes producteurs d'estérases dans lequel l'ingrédient actif comprend un ester acide aromatique d'un phénol ou d'un alcool aromatique, le phénol ou alcool aromatique étant suffisamment hydrosoluble pour conférer une action anti-microbienne et l'acide aromatique étant suffisamment hydrosoluble pour conférer une action anti-microbienne et/ou pour abaisser le pH de la sécrétion corporelle liquide à un niveau qui empêche au moins la croissance de micro-organismes dans les sécrétions corporelles liquides, une composition parfumée, et un véhicule pour l'ingredient actif et la composition parfumée.

4. Utilisation selon la revendication 1 ou 2, ou une composition selon revendication 3, dans laquelle une ou plusieurs des positions disponibles du noyau de benzène dudit phénol sont occupées par des groupes hydroxyle, aliphatique, benzénoïde ou hétérocyclique, et où ledit phénol est choisi de préférence parmi le phénol, les crésols, les xylénols, le thymol, le carbocrol, l'eugénol et l'isoeugénol.

5. Utilisation selon la revendication 1 ou 2, ou une composition selon la revendication 3, dans laquelle une ou plusieurs des positions disponibles du noyau de benzène de l'alcool aromatique sont occupées par des groupes hydroxyle, aliphatique, benzénoïde ou hétérocyclique, et où ledit alcool aromatique est choisi de préférence parmi l'alcool benzylique, l'alcool phényléthylique, l'alcool cinnamique et l'alcool anisique.

6. Utilisation selon la revendication 1, 2, 4 ou 5, ou une composition selon revendication 3, 4 ou 5, dans laquelle une ou plusieurs des positions disponibles du noyau de benzène dudit acide aromatique sont occupées par un groupe hydroxyle, aliphatique, benzénoïde ou hétérocyclique, et où ledit acide aromatique est choisi de préférence parmi l'acide benzoïque, l'acide salicylique, l'acide cinnamique, l'acide phénylacétique et l'acide anisique.

7. Utilisation selon la revendication 2, ou selon les revendications 4, 5 ou 6 qui y sont annexées, ou une composition selon revendication 3, 4, 5 ou 6, dans laquelle ledit véhicule comprend un alcool.

8. Utilisation ou une composition selon la revendication 7, dans laquelle ledit ester acide aromatique est présent dans une proportion de 5% - 50% dans ladite composition parfumée, et où ledit ester acide aromatique et ladite composition parfumée constituent environ 1% de ladite composition, ledit véhicule étant un excipient d'éthanol à 96% approximativement.

9. Utilisation selon la revendication 1, ou selon les revendications 4, 5 ou 6 annexées à celle-ci, ou une composition selon l'une quelconque des revendications 3 à 6, dans laquelle ledit ester acide aromatique est incorporé dans un véhicule qui comporte un alcool aqueux et/ou des pénétrants cutanés pour produire également une action dermatologique.

10. Utilisation ou une composition selon revendication 9, et comportant en outre des substances anti-inflammatoires et/ou des agents curatifs.